# EUROPEAN PATENT APPLICATION

(11) **EP 4 197 422 A1**
(43) Date of publication of application: **21.06.2023**
(21) Application number: 22214024.6
(22) Date of filing: 15.12.2022
(51) Int. Cl.: A61B 1/00, A61B 1/045, A61B 1/06, A61B 1/12, G02B 27/00

(54) **ENDOSCOPE, ENDOSCOPIC SYSTEM AND METHOD OF OPERATING THE ENDOSCOPIC SYSTEM**

(30) Priority: 15.12.2021 DE 102021133341
(71) Applicant: Karl Storz SE & Co. KG, 78532 Tuttlingen (DE)
(72) Inventor: Baum, Eckhart, 78532 Tuttlingen (DE); Schwarz, Peter, 78532 Tuttlingen (DE)
(74) Representative: Wimmer, Stephan

(57) **Abstract**

A method of operating an endoscopic system (10) comprising a light source (40), an endoscope (20) with a distal light entrance surface (23), an image sensor (25) capturing images through the light entrance surface (23) and a rinsing device (70) providing a rinsing fluid flow for rinsing the light entrance surface (23), the method comprising receiving (101; 106) a sensor signal from a sensor (31; 35; 36; 37; 76; 77) detecting a user input at a user interface (30), the user input starting a rinsing operation of the rinsing device (70), and, upon receipt of the sensor signal, changing (102, 107) an illumination mode of the light source (40) from a first illumination mode to a second illumination mode.

## Description

The present invention refers to a method of operating an endoscopic system, an endoscopic system and components thereof.

An image sensor usually comprises a large number of pixels arranged in a matrix. In each pixel, light impinging on the pixel produces an electric charge or another electrical signal. In a CCD sensor, all the pixels are light sensitive simultaneously, i.e. within the same period of time between a global reset signal and a global read signal. This is also known as a global shutter or global shutter mode.

In a CMOS sensor, pixels in different rows are light sensitive in different time intervals, wherein the time interval of each subsequent row is slightly later than that of the previous row. This is known as a rolling shutter or rolling shutter mode. When a fast moving object is captured, the rolling shutter can cause a heavily distorted image of the fast moving object. This is known as the rolling shutter effect.

In medical applications, rarely is an object moving fast, and the rolling shutter effect is not relevant.

However, in some applications, the light entrance interface at the distal end of an endoscope can be contaminated. Both solid and liquid debris can severely reduce the image quality. Therefore, some endoscopes provide the option to rinse the light entrance surface in situ.

Both a rinsing liquid flowing across the light entrance surface and rinsing liquid droplets or debris blown away by a rinsing gas are fast moving objects causing the rolling shutter effect in a CMOS sensor. Moreover, the bright illumination light causes bright reflections in liquid droplets on the distal light entrance surface, and these bright reflections are distorted by the rolling shutter effect. The resulting optical experience is perceived by medical personnel as a disturbing indication of a broken optical or electronic apparatus.

In US 2014-0171738 A1 (also published as US 9,801,530 B2), an endoscope apparatus having a CMOS type solid-state image pickup element and an image pickup control method are described. A fluid detection device detects a fluid operation based on an object region edged along a direction parallel to scan lines of the image pickup element. An exposure control device lengthens an exposure time if the fluid operation detection device detects the fluid operation. If an edging phenomenon is detected the driving method of an image pickup element is switched to a pseudo-global shutter method.

In US 2012 0059222 A1, an imaging device and a method of cleaning an illumination window of the imaging device are described. In response to an instruction to start emission of near-infrared light, saline is discharged through nozzles to clean light applying lenses and an imaging lens in order to avoid burning of contaminants on a light exit window at the distal end of an endoscope.

An object of the present invention is to provide an improved method of operating an endoscopic system, an improved endoscope, an improved rinsing device, an improved camera control unit and an improved endoscopic system.

This object is solved by the subject matters of the independent claims.

Further embodiments are described in the dependent claims.

Some embodiments of the present invention are based on the idea to change an illumination mode and optionally also a capturing mode when a sensor signal is received from a sensor detecting a user input starting a rinsing operation. For this purpose, some of the embodiments are based on the idea to detect a manual operation of a valve controlling a flow of the rinsing fluid or detect a pressure or a change of pressure or a flow or a change of flow of rinsing fluid.

In a method of operating an endoscopic system comprising a light source, an endoscope with a distal light entrance surface, an image sensor capturing images through the light entrance surface and a rinsing device providing a rinsing fluid flow for rinsing the light entrance surface, the method comprises receiving a sensor signal from a sensor detecting a user input at a user interface, the user input starting a rinsing operation of the rinsing device, and, upon receipt of the sensor signal, changing an illumination mode of the light source from a first illumination mode to a second illumination mode.

The endoscope can comprise an entirely rigid shaft or a shaft partially rigid and partially flexible or an entirely flexible shaft. The endoscope is in particular provided and configured for medical applications. In this case, the endoscope is in particular hermetically sealed and tolerates the temperature, the pressure and the atmosphere in an autoclave during steam sterilization. As an alternative, the endoscope can be provided and configured for non-medical, technical applications.

A lens or objective and the image sensor can be located immediately behind or downstream the light entrance surface, in particular at the distal end of the endoscope. As an alternative, the endoscope may comprise a number of rod lenses or other relay lenses relaying the image produced by the lens or objective to the proximal end of the endoscope. In the latter case, the image sensor can be located at the proximal end of the endoscope or in a camera or another separate device which can be coupled to the proximal end of the endoscope.

Illumination light provided by the light source and light emanating from an object and forming an image captured by the image sensor can be transmitted through the same light entrance surface and through the same optically transparent member but in the opposite direction. As an alternative, illumination light can be emitted through a different optically transparent member.

The light source can be part of the endoscope, in particular located at and/or integrated in the distal end of the shaft. As an alternative, the light source can be a separate device coupled to the endoscope, in particular to the proximal end of the endoscope, by a fiber optic or other light conducting cable. The light source can comprise a light emitting diode, a laser, a halogen lamp, a gas discharge lamp or any other lamp.

The rinsing device can comprise a rinsing fluid reservoir or can be coupled to a rinsing fluid reservoir. The rinsing fluid can be water or another rinsing liquid or air or another rinsing gas. The rinsing device in particular comprises a pump pressurizing and/or conveying the rinsing fluid. A hydraulic accumulator (a container partially filed with a compressed gas and partially filled with the liquid, in particular with a flexible membrane in between) may be located downstream the pump. If the rinsing fluid is a gas provided in a pressure vessel, no pump is required. However, a pump can be provided anyway, for example upstream the pressure vessel.

In particular, the sensor signal is received from a sensor mechanically or hydraulically or pneumatically coupled to the user interface. The user interface can comprise or consist of a button to be pressed manually, a lever to be flipped or pulled or swiveled manually, a slide to be moved or shifted manually, a knob to be turned or rotated manually for example. The user interface can be intended and provided and designed exclusively for controlling the rinsing operation. As an alternative, the user interface can be intended and provided and designed for controlling other processes or operations or procedures or functions of the endoscope system as well.

The user interface can be provided exclusively for starting a rinsing operation. In this case, the endoscopic system can be provided and configured to stop the rinsing operation after a predetermined period of time. As an alternative, the user interface is further provided and configured for receiving a user input arbitrarily ending the rinsing operation. This user input can consist in releasing the button.

In particular, the intensity or power of illumination light is constant in the first illumination mode. The second illumination mode can differ from the first illumination mode in the intensity or power of illumination light. As an alternative or in addition, the second illumination mode may differ from the first illumination mode in color or spectral properties of the illumination light. As a further alternatively or in addition, the second illumination mode can differ from the first illumination mode in the time-dependent pattern of intensity, power, color, and/or spectral characteristics.

Receiving a sensor signal from a sensor detecting a user input starting a rinsing operation can be both a simple and easily implemented and a particularly reliable solution.

In a method as described herein, the image sensor in particular provides a rolling shutter mode, wherein image pixels in different regions of the image sensor are sensitive to light within non-identical periods of time, wherein within a vertical blanking interval all pixels are sensitive to light and in each pixel received light contributes to an image captured by the image sensor.

The rolling shutter mode is prevalent in CMOS image sensors. The electrical signals representing the light collected in each of the pixels are read from the pixels row by row. Only pixels in the same row are sensitive to light and producing signals representing light collected (more or less) within the same period of time. The periods of time in which the pixels of consecutive rows are sensitive to light usually overlap or heavily overlap but are not identical but shifted or offset against each other.

In a method as it is described herein, receiving the sensor signal in particular comprises receiving a sensor signal from a sensor detecting a manual operation of a valve controlling flow of the rinsing fluid.

The valve can be located in or at the endoscope, in particular in or at the endoscope's proximal end, in or at the rinsing device or anywhere else in the endoscopic system. The valve is controlled manually, in particular by pressing a button, swiveling a lever, moving a slider or turning a knob, wherein the button or lever or slider or knob is mechanically coupled to a valve member controlling the flow of the rinsing fluid.

In a method as it is described herein, receiving a sensor signal in particular comprises receiving a sensor signal from a hall sensor or a switch or another sensor detecting a position of a valve member of the valve.

A hall sensor can detect the magnetic field of a permanent magnet integrated with or coupled to the valve member. As an alternative, the hall sensor can detect a magnetic field produced by a stationary permanent magnet and altered by the valve member.

As an alternative, the sensor can be an electrical switch coupled to the valve member. The switch closes a circuit when the valve is open, i.e. rinsing fluid flows, and opens the circuit when the valve is closed, i.e. no fluid flows, or vice versa.

As an alternative, the sensor can be a photoelectric sensor detecting the position of the valve member.

In a method as described herein, receiving the sensor signal in particular comprises receiving a sensor signal from a sensor detecting at least one of a pressure or a flow or a change of pressure or a change of flow of the rinsing fluid.

When the rinsing fluid is a rinsing liquid, a sensor detecting a flow or a change of flow of the rinsing liquid can be located virtually anywhere between a reservoir providing the rinsing liquid and a fluid outlet at the distal end of the endoscope.

When a pressure sensor signal is received from a pressure sensor, this pressure sensor is, in particular, located between the valve and the fluid outlet at the distal end of the endoscope. However, starting (and stopping) a rinsing operation, i.e. flow of the rinsing fluid, effects the pressure upstream the valve, too, at least in a short term, and at least when the rinsing fluid is a rinsing liquid.

Detecting the pressure or the flow of the rinsing fluid is a way of detecting a user input at the manually operated valve.

In a method as described herein, receiving the sensor signal in particular comprises receiving the sensor signal from a sensor in the endoscope or in the rinsing device.

Each of the examples mentioned above, in particular a sensor detecting the status of a user interface, a sensor detecting a pressure and a sensor detecting a flow, can be located in or at the endoscope as well as in or at the rinsing device.

In a method as described, spectral characteristics of illumination light provided by the light source are, in particular, equal in the first and second illumination light modes, wherein power or intensity or time dependence of power or intensity of illumination light provided by the light source is different in the first and second illumination modes.

In a method as described herein, in the second illumination mode, power or intensity of light provided by the light source is in particular higher within a vertical blanking interval of the image sensor than outside the vertical blanking interval.

In a method as described herein, in the second illumination mode the energy of light provided by the light source within a vertical blanking interval of the image sensor is in particular higher than the energy of light provided by the light source outside the vertical blanking interval.

In a method as described herein, in the second illumination mode the light source in particular provides light within a vertical blanking interval of the image sensor only.

In a method as described herein, in the second operating mode the intensity of light provided by the light source is in particular reduced.

In particular, the intensity of light provided in the second illumination mode is between 10% and 20%, in particular 15%, of the intensity provided in the first illumination mode.

When the intensity is reduced, the exposure control increases the exposure time. An increased exposure time causes motion blur of all artifacts produced by the rolling shutter effect. When the intensity of illumination light is further reduced beyond what can be compensated by prolonged exposure time, the image gets darker. This reduces the brightness of the artifacts produced by the rolling shutter effect and the irritation of medical personnel.

In a method as described herein, upon receipt of the sensor signal, in particular a capturing mode of the image sensor is changed from a first capturing mode to a second capturing mode.

In particular, the second capturing mode is different from the first capturing mode. For example, the exposure time, the sensitivity or electronic gain, the frame rate and/or the resolution can be different in the first and second capturing modes.

In a method as described herein, in the second operating mode the exposure time is in particular set to a maximum value.

The exposure time is the exposure time of each single pixel. The exposure time is the time between a reset signal resetting the pixel and a read signal triggering readout of the pixel. The maximum value of the exposure time is given by the frame rate. The maximum value of the exposure time is provided when the reset signal immediately (as fast as technically possible) follows the read signal.

A method as described herein in particular further comprises receiving a further signal indicating an end of the rinsing operation from the sensor or from another sensor and, upon receipt of the further signal, changing the illumination mode of the light source from the second illumination mode to the first illumination mode.

In the examples described above, the signal and the further signal are in particular received from the same sensor. For example, the signal is an increase of a voltage caused by closing of a circuit by a switch mechanically coupled to a manually operated user interface or to a manually moved valve member, wherein the further signal is a decrease of the voltage caused by an opening of the circuit caused by an opening of the switch. As an alternative, the further signal is received from another sensor, for example detecting at the same user interface or at another user interface a user input requesting the end of the rinsing operation.

When the illumination mode is changed from the second illumination mode to the first illumination mode, the capturing mode can be changed simultaneously from the second capturing mode to the first capturing mode.

In a method as described herein, the rinsing operation comprises a gas flowing at the distal light entrance surface after a rinsing liquid flew at the distal light entrance surface, and receiving the further signal comprises receiving a further signal indicating that the gas flow is terminated.

A rinsing operation comprising rinsing of the distal light entrance surface by a rinsing liquid within a first period of time, and, thereafter, drying of the distal light entrance surface (or blowing away liquid droplets) by a gas flow within a consecutive second period of time can be advantageous in many applications. After the gas flow is terminated, there can be no fast moving objects on the distal light entrance surface, and, thus, no rolling shutter effect is to be expected.

In a method as described herein, the operating mode of the light source is in particular gradually changed back from the second illumination mode to the first illumination mode.

For example, the intensity of illumination light is slowly (within a fraction of a second or within a second or within a few seconds) increased to the original level of the first illumination mode, or the intensity or power or energy of illumination light provided in the intervals between the vertical blanking intervals is slowly increased.

An endoscope comprises a shaft; a light entrance surface at a distal end of the shaft; a rinsing fluid duct in the shaft conducting a rinsing fluid from the proximal end of the endoscope to the light entrance surface for rinsing the light entrance surface; at least one of a pressure sensor for detecting a pressure or a change of pressure in the rinsing fluid duct and a flow sensor for the detection of a fluid flow or a change of fluid flow in the rinsing fluid duct and a user interface sensor for directly detecting a user input at a user interface, the user input requesting rinsing of the light entrance surface; and a sensor signal output configured to provide a signal representing the pressure or the change of pressure detected by the pressure sensor or the flow or the change of flow detected by the flow sensor or the user input detected by the user interface sensor, respectively.

An endoscope as described herein is in particular configured to be part of a system conducting a method as described herein.

In particular, the endoscope provides above-described features, characteristics and/or functions.

A rinsing device comprises a pressure source providing pressure for conveying rinsing fluid; a rinsing fluid output fluidly coupled to the pressure source for providing rinsing fluid to an endoscope; at least one of a pressure sensor for the detection of a pressure or a change of pressure of rinsing fluid and a flow sensor for detection of a flow or a change of flow of rinsing fluid and a user interface sensor for directly detecting a user input at a user interface, the user input requesting rinsing of a light entrance surface of the endoscope; and a sensor signal output configured to provide a signal representing the pressure or the change of pressure detected by the pressure sensor or the flow or the change of flow detected by the flow sensor or the user input detected by the user interface sensor, respectively.

In particular, the pressure source comprises a pump or a pressure vessel.

A rinsing device as described herein is in particular configured to be part of a system conducting a method as described herein.

In particular, the rinsing device provides above-described features, characteristics and/or functions.

A camera control unit comprises an image signal input for receiving an image signal representing an image captured by an image sensor of an endoscope or a camera coupled to an endoscope; a sensor signal input for receiving a sensor signal indicating at least one of a pressure or a change of pressure or a flow or a change of flow of a rinsing fluid and a user interface sensor directly detecting a user input requesting rinsing of a light entrance surface of an endoscope; and a control output for providing a control signal changing an illumination mode of a light source from a first illumination mode to a second illumination mode when a sensor signal indicating a rinsing operation is received at the sensor signal input.

A camera control unit as described herein is in particular configured to be part of a system conducting a method as described herein.

In particular, the camera control unit provides above-described features, characteristics and/or functions.

An endoscopic system comprises an endoscope with a shaft, a light entrance surface at a distal end of the shaft and a rinsing fluid duct in the shaft for conducting a rinsing fluid from the proximal end of the endoscope to the light entrance surface for rinsing the light entrance surface; a light source for providing illumination light for the endoscope; at least one of a pressure sensor for detection of a pressure or change of pressure of rinsing fluid and a flow sensor for detection of a flow or a change of flow of rinsing fluid and a user interface sensor for detecting a user input at a user interface, the user input requesting rinsing of the light entrance surface; and a controller coupled to the pressure or flow sensor or user interface sensor, respectively, and to the light source, for changing an illumination mode of the light source from a first illumination mode to a second illumination mode when a sensor signal from the pressure sensor or flow sensor or user interface sensor, respectively, indicates a rinsing operation.

An endoscopic system as described herein is in particular configured to conduct a method as described herein.

In particular, the endoscopic system provides above-described features, characteristics and/or functions.

In particular, the light source can be located in the endoscope or otherwise integrated with the endoscope or can be a separate device coupled to the endoscope by a fiber optic cable or another light conducting cable. The pressure sensor or flow sensor or user interface sensor, respectively, can be located in the endoscope or otherwise integrated with the endoscope or can be located at or in a rinsing device providing the rinsing fluid flow. Furthermore, the user interface sensor can be part of a user interface which can be a separate device, for example a foot switch.

### Short description of the figures

Embodiments will be described below with reference to the enclosed figures.
- Figure 1: shows a schematic representation of an endoscopic system;
- Figure 2: shows a schematic representation of another endoscopic system;
- Figure 3: shows a schematic representation of another endoscopic system;
- Figure 4: shows a schematic representation of another endoscopic system;
- Figure 5: shows a schematic representation of another endoscopic system;
- Figure 6: shows a schematic representation of another endoscopic system;
- Figure 7: shows a schematic representation of a time dependence of an intensity of illumination light;
- Figure 8: shows a schematic representation of another time dependence of an intensity of illumination light;
- Figure 9: shows a schematic flowchart of a method of operating an endoscopic system.

### Description of embodiments

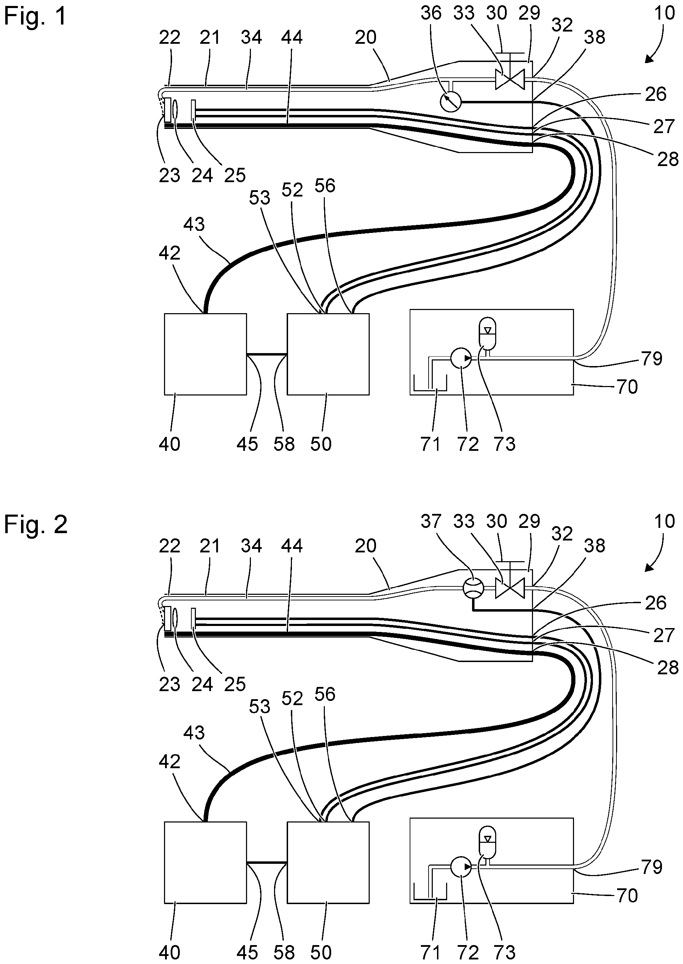

Figure 1 shows a schematic representation of and endoscopic system 10 comprising an endoscope 20, a light source 40, a camera control unit 50 and a rinsing device 70. The endoscopic system 10 can be provided and configured for medical purposes. In this case, the endoscope 20 is in particular hermetically sealed and tolerates the temperature, the pressure and the atmosphere in an autoclave during steam sterilization. As an alternative, the endoscopic system 10 can be provided and configured for non-medical, technical applications.

The endoscope 20 comprises a long and thin shaft 21. The shaft 21 can be entirely rigid or partially rigid and partially flexible or entirely flexible. The shaft 21 is provided and configured to be inserted into a cavity in the body of a human or animal patient.

At a distal end 22 of the shaft 21, light emanating from an object can enter the endoscope 20 through a distal light entrance surface 23. A lens 24 downstream the distal light entrance surface 23 forms a real image in an image sensor 25. In the example shown in Figure 1, the image sensor 25 is located at the distal end 22 of the endoscope. As an alternative, the real image produced by the lens 24 can be transferred to the proximal end 29 of the endoscope 20 by a relay lens system or by a bundle of optical fibers. In this case, the image sensor 25 can be located at the proximal end 29 of the endoscope 20 or in a camera or another device optically coupled to the proximal end 29 of the endoscope.

The endoscope 20 comprises an image sensor control input 26 receiving an image sensor control signal from the camera control unit 50. The image sensor control input 26 is coupled to the image sensor 25. Parameters like the frame rate, the size of the image, the exposure time, the sensitivity or electronic gain to be applied by the image sensor 25 itself and timing information can be part of the image sensor control signal.

Furthermore, the endoscope 20 comprises an image signal output 27 providing an analog or digital image signal generated by the image sensor 25 and representing a real image produced by the lens 24 and captured by the image sensor 25. In particular and in the present example, the image signal provided at the image signal output 27 is transferred to the camera control unit 50.

In particular, both the image sensor control input 26 and the image signal output 27 are situated at the proximal end 29 of the endoscope 20. The image sensor control input 26 and the image signal output 27 can be integrated in one connector transferring both the image sensor control signal and the image signal in opposite directions.

Furthermore, the endoscope 20 comprises an illumination light input 28 receiving illumination light from the light source 40. Illumination light received at the illumination light input 28 is guided by one or more optical fibers 44 to the distal end 22 of the endoscope and radiated on an object to be viewed. In the example shown in Figure 1, the illumination light input 28 is situated at the proximal end 29 of the endoscope 20.

Furthermore, the endoscope 20 comprises a user interface 30 at the proximal end 29. In the example shown in Figure 1, the user interface 30 comprises a button which can be manually pressed. A rinsing fluid input 32 is provided at the proximal end 29 of the endoscope 20. A valve 33 connects the rinsing fluid input 32 to a rinsing fluid duct 34. The rinsing fluid duct 34 connects the valve 33 with a rinsing fluid outlet at the distal end 22 of the endoscope 20. The user interface 30 is coupled to a valve member of the valve 33. Rinsing fluid flow to the distal end 22 of the endoscope can be controlled by manual operation of the user interface 30.

A pressure sensor 36 is coupled to the rinsing fluid duct 34 downstream the valve 33 and detects the pressure in the rinsing fluid duct 34. The pressure sensor 36 is coupled to a sensor signal output 38 at the proximal end 29 of the endoscope 20. A sensor signal produced by the pressure sensor 36 and representing the pressure in the rinsing fluid duct 34 detected by the pressure sensor 36 or a change of the pressure is provided at the sensor signal output 38. The senor signal output 38 is coupled to the camera control unit 50.

In Figure 1, the sensor signal output 38 is a separate connector. As an alternative, the sensor signal output 38 can be integrated with the image sensor control input 26 and/or with the image signal output 27.

The light source 40 provides an illumination light output 42 coupled to the illumination light input 28 by a light guiding cable 43. Illumination light produced by the light source 40 and provided at the illumination light output 42 is transferred to the illumination light input 28 of the endoscope through the light guiding cable 43. In the endoscope 20, the optical fibers 44 guide the illumination light to the distal end 22 of the endoscope 20 where it exits the endoscope 20 and illuminates an object to be viewed.

The light source 40 comprises a control input 45 receiving a control signal from the camera control unit 50.

The camera control unit 50 provides an image sensor control output 52 coupled to the image sensor control input 26 of the endoscope 20, an image signal input 53 coupled to the image signal output 27 of the endoscope 20 and a sensor signal input 56 coupled to the sensor signal output 38 of the endoscope 20. The sensor signal input 56 can be integrated in one connector together with the image sensor control output 52 and/or with the image signal input 53. In this case, the sensor signals produced by the pressure sensor 36 and/or image sensor control signals and/or image signals can be transferred via the same wires or fibers.

Furthermore, the camera control unit 50 comprises a control output 58 coupled to the control input 45 of the light source.

The rinsing device 70 comprises a rinsing fluid reservoir 71 supplying rinsing fluid. In the example shown in Figure 1, the rinsing fluid reservoir 71 is a rinsing liquid reservoir and the rinsing device 70 further comprises a rinsing fluid pump 72 drawing rinsing fluid from the rinsing fluid reservoir 71, conveying and pressurizing the rinsing fluid, and a pressure reservoir 73. The rinsing device 70 can comprise further components not shown in Figure 1, in particular a pressure sensor detecting the pressure produced by the rinsing fluid pump 72, a controller controlling the rinsing fluid pump 72 depending on the pressure detected by the pressure sensor and a user interface.

As an alternative, the rinsing fluid reservoir 71 can be a rinsing gas reservoir, in particular a pressure vessel containing pressurized gas, for example carbon dioxide, nitrogen or air. In this case, the rinsing fluid pump 72 and the pressure reservoir 73 can be replaced by a pressure reducer reducing the pressure of the gas from the gas vessel to a predetermined value.

As a further alternative, the rinsing device 70 can comprise both a rinsing fluid reservoir 71 together with a rinsing fluid pump 72 and a pressure reservoir 73 and a gas vessel providing a rinsing gas together with a pressure reducer. In this case, the rinsing device 70 can comprise a controller controlling a predetermined procedure or sequence. In particular, the procedure comprises providing a rinsing liquid within a first period of time and, thereafter, providing a rinsing gas in a second period of time, the rinsing gas blowing away or drying rinsing liquid droplets or a rinsing liquid film on the distal light entrance surface 23 of the endoscope.

The rinsing device 70 comprises a rinsing fluid output 79 coupled to the rinsing fluid input 32 of the endoscope by a flexible tube or hose. If the rinsing device 70 provides both a rinsing liquid and a rinsing gas, both can be provided at two different rinsing fluid outputs 79 and conducted to the endoscope 20 by two distinct flexible tubes or hoses. As an alternative, both the rinsing liquid and the rinsing gas can be provided at the same rinsing fluid output 79 and conducted to the endoscope 20 by the same flexible tube or hose.

A user can initiate a rinsing operation by opening the valve 33 by a manual operation of the user interface 30, in particular pressing a push button. When the valve 33 is open, rinsing fluid provided by the rinsing device 70 flows through the rinsing fluid duct 34 to the distal end 22 of the endoscope 20 and rinses the distal light entrance surface 23 of the endoscope 20. The user can stop the rinsing fluid flow by another manual operation of the user interface 30, for example releasing said push button, thereby closing the valve 33. As an alternative, the valve 33 automatically closes after a predetermined period of time set to a value usually sufficient to remove debris from the distal light entrance surface 23.

If the rinsing device 70 provides both a rinsing liquid and a rinsing gas, two valves 33 can be provided and can be manually operated separately, one valve controlling the rinsing liquid flow, and the other valve controlling the rinsing gas flow. As an alternative, merely the valve 33 controlling the rinsing liquid flow is directly controlled by a manual operation of the user interface 30, and a second valve controlling the rinsing gas flow is automatically open and allows a rinsing gas flow during a predetermined period of time after the end of the rinsing liquid flow. As a further alternative, the user input at the user interface 30 merely opens the valve 33 controlling the rinsing liquid flow but a controller closes the valve 33 thereby stopping the rinsing liquid flow after a first predetermined period of time and, thereafter opens a second valve controlling the rinsing gas flow for a second predetermined period of time.

When a rinsing fluid is flowing in the rinsing fluid duct 34, the pressure in the rinsing fluid duct 34 is different from, in particular higher than in the situation of no rinsing fluid flow. In particular, due to the flow resistance of the rinsing fluid duct 34 the pressure of the rinsing fluid immediately downstream the valve 33 is higher when the valve 33 is open. Immediately after opening the valve 33, the pressure increases and immediately after closing the valve 33, the pressure decreases. Therefore, the pressure of the rinsing fluid in the rinsing fluid duct 34 detected by the pressure sensor 36 indicates the beginning and the end of a rinsing operation.

The camera control unit 50 receives, at its sensor signal input 56, the sensor signal from the pressure sensor 36. The camera control unit 50 controls the light source 40 by a control signal provided at the control output 58 of the camera control unit 50 and received by the light source 40 at its control input 45.

During normal use of the endoscopic system 10, i.e. when there is no rinsing operation, the light source 40 is in a first illumination mode. As an option, details of the first illumination mode, in particular power or intensity and the spectral characteristics (like color temperature) can be controlled by a user, in particular at a user interface at the light source 40.

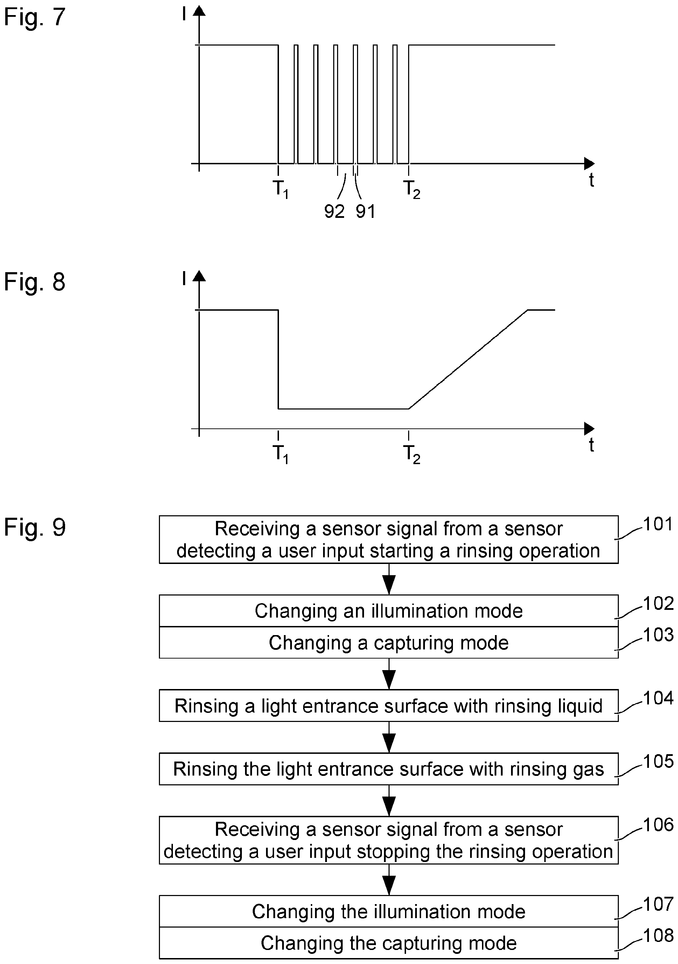

At the beginning of a rinsing operation, the light source 40 is set to a second illumination mode different from the first illumination mode. As described in more detail below with reference to Figures 7 and 8, the second illumination mode can differ from the first illumination mode in the intensity or power of the illumination light or in the time dependence of the intensity or power, for example. When the end of the rinsing operation is detected, the light source 40 instantaneously or gradually returns to the first illumination mode.

The rinsing device 70 can comprise a valve at its rinsing fluid output 79 preventing leakage or unintended output of rinsing fluid from the rinsing fluid output 79 when no tube or hose is connected to the rinsing fluid output 79 or when no endoscope 20 is connected to the downstream end of the tube or hose.

During rinsing operation, the camera control unit 50 optionally not only controls a second illumination mode of the light source 40 but simultaneously a second capturing mode of the image sensor 25 different from a first capturing mode controlled when there is no rinsing operation. In particular, the exposure time of each single image captured by the image sensor 25 is set to a maximum value in the second capturing mode. When the exposure time is maximum, the reset signal immediately follows on the read signal for each pixel. If the image sensor 25 is a CMOS-sensor providing a rolling shutter, there is a well-defined period of time called the vertical blanking interval during which all the pixels are sensitive to light simultaneously.

Figure 2 shows a schematic representation of another endoscopic system 10 similar to the endoscopic system described with reference to Figure 1 with regard to many features, characteristics and functions. Hereinafter, differences of the endoscopic system 10 shown in Figure 2 from the endoscopic system described above with reference to Figure 1 are described.

In the endoscopic system 10 shown in Figure 2, the endoscope 20 comprises a flow sensor 37 in the rinsing fluid duct 34. The flow sensor 37 detects the mass flow or the volume flow in the rinsing fluid duct 34 and provides a sensor signal representing the detected mass flow or volume flow of the rinsing fluid in the rinsing fluid duct 34. The camera control unit 50 receives the sensor signal from the flow sensor 37 and controls the light source 40. When the rinsing fluid flow detected by the flow sensor 37 and represented by the sensor signal increases and, thus, indicates the beginning of a rinsing operation, the light source 40 is set to the second illumination mode. When the rinsing fluid flow detected by the flow sensor 37 and represented by the sensor signal is decreasing and, thus, indicates an end of the rinsing operation, the light source 40 is instantaneously or gradually reset to the first illumination mode.

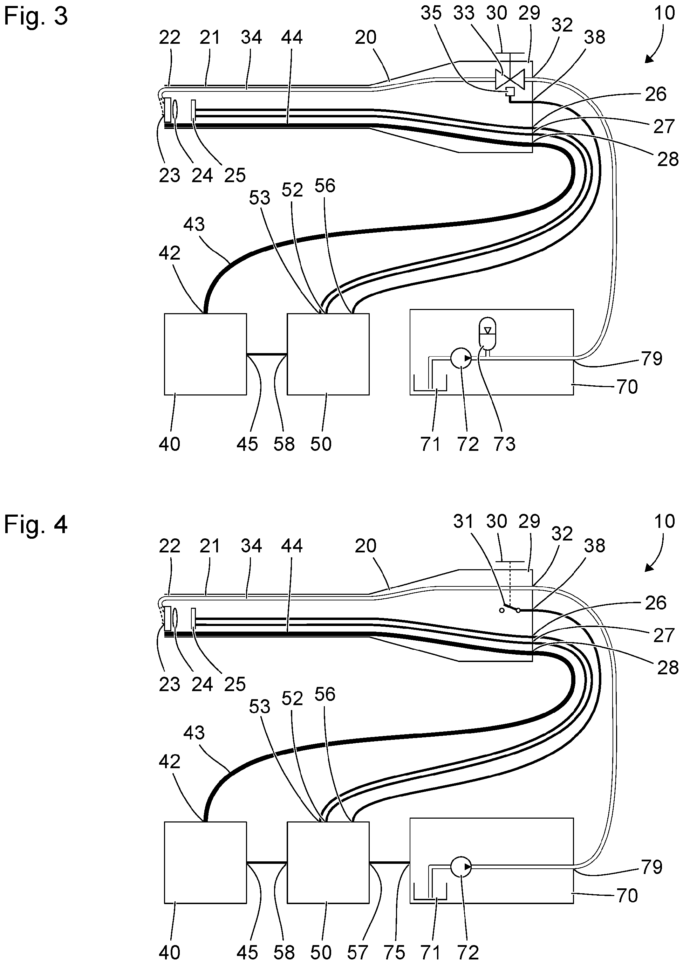

Figure 3 shows a schematic representation of another endoscopic system 10 similar to the endoscopic systems described with reference to Figures 1 and 2 with regard to many features, characteristics and functions. Hereinafter, differences of the endoscopic system 10 shown in Figure 3 from the endoscopic systems described above with reference to Figures 1 and 2 are described.

In the endoscopic system 10 shown in Figure 3, the endoscope 20 comprises a hall sensor 35 located near the valve 33. The hall sensor 35 detects the magnetic field produced by a permanent magnetic valve member of the valve 33 or the magnetic field of a permanent magnet mechanically coupled to the valve member of the valve 33 or the magnetic field produced by a stationary permanent magnet and altered by the valve member of the valve 33. The magnetic field detected by the hall sensor 35 depends on the (translational or rotational) position of the valve member of the valve 33 and indicates whether the valve 33 is closed or open. Thus, the sensor signal provided by the hall sensor 35 and received at the sensor signal input 56 of the camera control unit 50 indicates the closed or open status of the valve 33 and, thus, the beginning and the end of rinsing operation.

Similar to the embodiments described above with reference to Figures 1 and 2, the light source 40 is set to the second illumination mode at the beginning of a rinsing operation and instantaneously or gradually reset to the first illumination mode at the end of a rinsing operation.

Figure 4 shows a schematic representation of another endoscopic system 10 similar to the endoscopic systems described with reference to Figures 1 through 3 with regard to many features, characteristics and functions. Hereinafter, differences of the endoscopic system 10 shown in Figure 4 from the endoscopic systems described above with reference to Figures 1 through 3 are described.

In the endoscopic system 10 shown in Figure 4, the endoscope 20 comprises a user interface sensor 31 detecting a manual input at the user interface 30. In the example shown in Figure 4, the user interface sensor 31 is an electrical switch and the user interface 30 is a push button. When the push button 30 is manually pressed, the switch 31 closes a circuit, thereby for example altering the voltage at a signal line. This sensor signal is received at the sensor signal input 56 of the camera control unit 50.

The camera control unit 50 comprises a control output 57 coupled to a control input 75 of the rinsing device 70. When the camera control unit 50 receives a sensor signal indicating a user input at the user interface 30, the camera control unit 50 controls the light source 40 to switch from the first illumination mode to a second illumination mode and controls the rinsing device 70 to perform a rinsing operation, in particular the rinsing fluid pump 72 to convey rinsing fluid. When the push button 30 is released or after a predetermined period of time, the camera control unit 50 controls the rinsing device 70 to stop the rinsing operation, in particular the rinsing fluid pump 72 to stop conveying rinsing fluid, and the light source 40 to switch back to the first illumination mode.

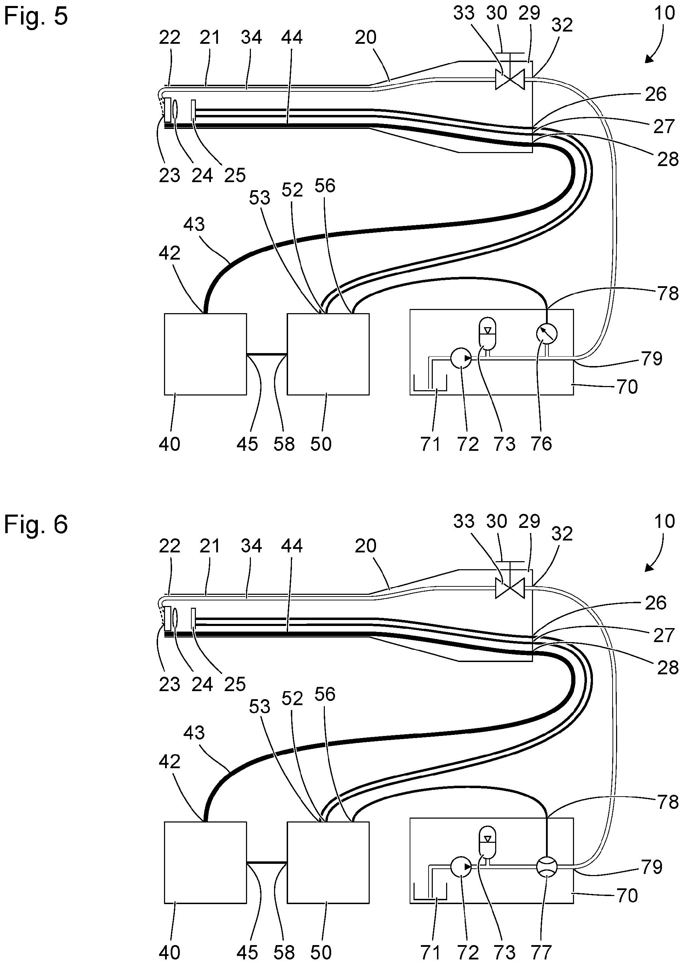

Figure 5 shows a schematic representation of another endoscopic system 10 similar to the endoscopic systems described with reference to Figures 1 through 4 with regard to many features, characteristics and functions. Hereinafter, differences of the endoscopic system 10 shown in Figure 5 from the endoscopic systems described above with reference to Figures 1 through 4 are described.

The endoscopic system 10 shown in Figure 5 is similar to the endoscopic system described above with reference to Figure 1, but the pressure sensor 36 in the endoscope 20 is replaced by a pressure sensor 76 in the rinsing device 70. The sensor signal input 56 of the camera control unit 50 is connected to a sensor signal output 78 of the rinsing device 70 providing the sensor signal generated by the pressure sensor 76.

Due to the flow resistance of the duct between the pressure reservoir 73 and the pressure sensor 76, the pressure sensor 76 detects a (small) decrease of rinsing fluid pressure when the valve 33 in the endoscope 20 is open end rinsing fluid is effluent from the distal end 22 of the endoscope 20. Therefore, a (small) decrease of pressure represented by the sensor signal received by the camera control unit indicates the beginning of a rinsing operation and a (small) increase of pressure indicates the end of the rinsing operation. The camera control unit can control the illumination mode of the light source 40 and the capturing mode of the image sensor 25 dependent on the sensor signal received from the pressure sensor 76.

Figure 6 shows a schematic representation of another endoscopic system 10 similar to the endoscopic systems described with reference to Figures 1 through 5 with regard to many features, characteristics and functions. Hereinafter, differences of the endoscopic system 10 shown in Figure 6 from the endoscopic systems described above with reference to Figures 1 through 5 are described.

The endoscopic system 10 shown in Figure 6 is similar to the endoscopic system described above with reference to Figure 2, but the flow sensor 37 in the endoscope 20 is replaced by a flow sensor 77 in the rinsing device 70. Similar to the endoscopic system 10 described above with reference to Figure 5, the sensor signal input 56 of the camera control unit 50 is coupled to a sensor signal output 78 of the rinsing device 70 providing the sensor signal produced by the flow sensor 77. A rinsing fluid flow detected by the flow sensor 77 indicates an ongoing rinsing operation. Therefore, the camera control unit 50 can control the illumination mode of the light source 40 and the capturing mode of the image sensor 25 dependent on the sensor signal received from the flow sensor 77.

In modifications of each of the embodiments described above with reference to Figures 1 through 6, the light source 40 can be partially or fully integrated in the endoscope 20. In particular, one or more light emitting diodes can be located at the distal end 22 of the endoscope or at the proximal end 29 of the endoscope 20 and coupled to the distal end 22 by one or more optical fibers. Furthermore, one or more semiconductor lasers or other lasers can be located in the endoscope 20 and provide excitation light exciting fluorescence in tissue for diagnostic purposes.

In further modifications of each of the embodiments described above with reference to Figures 1 through 6, a part of or the entire camera control unit 50 can be integrated in the endoscope 20. In particular, the above described control of the illumination mode of the light source 40 and of the capture mode of the image sensor 25 can be provided by a controller located in the endoscope 20.

Figure 7 shows a schematic diagram of time dependence of intensity I. The abscissa is assigned to the time t and the ordinate is assigned to the intensity I. Before a time T₁, the entire endoscopic system and in particular the image sensor is in a viewing mode or first capturing mode for viewing an object. Exposure is controlled by a variation of the electronic gain or the sensitivity of the pixels and/or a variation of the exposure time which is the time lag between the reset signal and the read signal for each single pixel. The intensity I of illumination light is constant, for example maximum or set to a value selected by the user.

At the time T₁, a sensor signal from a pressure sensor or a flow sensor or a hall sensor or any other sensor detecting a user input at a user interface is received where the user input starts a rinsing operation. The image sensor is switched to a second capturing mode providing a maximum exposure time. Within a vertical blanking interval, all pixels are sensitive to light, i.e. light received in each and every pixel contributes to the image captured. Simultaneously, the light source is switched to a second illumination mode. In the second illumination mode, illumination light is provided in the vertical blanking interval 91 only. No illumination light is provided in the interval 92 between two consecutive vertical blanking intervals 91. This produces the so-called pseudo global shutter mode.

At the time T₂, the sensor signal indicating the end of a rinsing operation is received. This causes switching back to the first capturing mode and the first illumination mode.

Figure 8 shows a schematic diagram of an alternative time dependence of the intensity I. This embodiment differs from the embodiment described above with reference to Figure 7 in two aspects.

The first difference between the embodiment shown in Figure 8 from the embodiment described above with reference to Figure 7 is that the intensity I is constant but non-zero in the second illumination mode between times T₁ and T₂. For example, the intensity is reduced to about 15% of the intensity in the first illumination mode. In this embodiment, controlling a second capturing mode is not required. Rather, exposure control of the camera control unit or of the image sensor automatically increases the exposure time due to the lower illumination. The increase of exposure time increases motion blur thereby reducing the irritating experience of the rolling shutter effect.

The second difference between the embodiment shown in Figure 8 and the embodiment shown in Figure 7 is that there is no instantaneous return to the first illumination mode. Rather, the light source gradually or slowly returns to the first illumination mode, and the intensity gradually increases to the original value. This can improve the user experience, in particular produce a smooth experience without abrupt changes in the image quality.

A gradual return to the first illumination mode and to the first capturing mode after the time T₂ can be implemented in the embodiment described above with reference to Fig. 7, too.

Figure 9 shows a schematic flowchart of a method of operating an endoscopic system. The method can be applied to one of the endoscopic systems described above with reference to Figures 1 to 6 or to another endoscopic system different from the endoscopic systems described above with reference to Figures 1 to 6. Therefore, hereinafter reference numerals of the embodiments described above with reference to Figures 1 through 6 are used as examples only.

In a first step 101, the sensor signal is received from a sensor 31, 35, 36, 37, 76, 77 detecting a user input starting a rinsing operation. The sensor can be coupled to a user interface 30 mechanically, like a switch 31 operated by a push button, wherein the push button is the user interface 30. As an alternative, the sensor can be coupled to the user interface magnetically, like a hall sensor 35 detecting the position or the change of position of a valve member which is directly coupled to the user interface. As a further alternative, the sensor can be coupled to the user interface 30 hydraulically or pneumatically, wherein the user interface 30 directly alters a rinsing fluid flow and the sensor 36, 37, 76, 77 detects the resulting change of rinsing fluid flow or rinsing fluid pressure.

When a sensor signal indicating a user input starting a rinsing operation is received, an illumination mode is changed 102 from a first illumination mode to a second illumination mode and a capturing mode is changed 103 from a first capturing mode to a second capturing mode. Examples of first and second illumination modes and first and second capturing modes are described above with reference to Figures 7 and 8.

Furthermore, started by the user input at a user interface, a light entrance surface 23 is rinsed with rinsing liquid. The light entrance surface 23 can be rinsed with rinsing liquid during a predetermined period of time or as long as the user wishes.

After rinsing 104 the light entrance surface with rinsing liquid, the light entrance surface 23 is optionally rinsed 105 with rinsing gas drying a rinsing liquid film on the light entrance surface 23 or blowing away rinsing liquid droplets. The rinsing gas can rinse 105 the light entrance surface 23 within a predetermined period of time or as long as a user wishes.

When the rinsing operation comprising rinsing 104 with rinsing liquid and optionally also rinsing 105 with rinsing gas is terminated, a sensor signal is received 106 from a sensor 31, 35, 36, 37, 76, 77 detecting a user input stopping the rinsing operation. The user input stopping the rinsing operation can comprise releasing a button, for example. As an alternative, the signal from a controller controlling the rinsing operation and ending the rinsing operation can be received or a signal from a sensor detecting the end of the rinsing operation (regardless of whether it is caused by a controller or by a user input) can be received.

When the sensor signal indicating the user input stopping the rinsing operation or indicating an end of the rinsing operation is received 106, the illumination mode is changed 107 back to the first illumination mode and the capturing mode is changed 108 back to the first capturing mode.

The embodiments described above with reference to Figures 1 through 8 are in particular provided and configured to perform or conduct the method described above with reference to Figure 9. As an alternative, each of the embodiments described above with reference to Figures 1 through 8 can be provided and configured to conduct or perform a method different from the method described above with reference to Figure 9.

### List of reference numerals

- **10**: **endoscopic system**
- **20**: **endoscope**
- 21: shaft of the endoscope 20
- 22: distal end of the shaft 21
- 23: distal light entrance surface at the distal end 22 of the shaft 21
- 24: lens of the endoscope 20
- 25: image sensor of the endoscope 20
- 26: image sensor control input of the endoscope 20
- 27: image signal output of the endoscope 20
- 28: illumination light input of the endoscope 20
- 29: proximal end of the endoscope 20
- **30**: **user interface** at the endoscope 20
- 31: user interface sensor at the user interface 30
- 32: rinsing fluid input of the endoscope 20
- 33: valve of the endoscope 20, controlling rinsing fluid flow
- 34: rinsing fluid duct of the endoscope 20
- 35: hall sensor at the valve 33
- 36: pressure sensor at the rinsing fluid duct 34
- 37: flow sensor at the rinsing fluid duct 34
- 38: sensor signal output of the endoscope 20
- **40**: **light source**
- 42: illumination light output of the light source 40
- 43: light guiding cable
- 44: optical fibers within the endoscope 20
- 45: control input of the light source 40
- **50**: **camera control unit**
- 52: image sensor control output
- 53: image signal input of the camera control unit 50
- 56: sensor signal input of the camera control unit 50
- 57: control output of the camera control unit 50
- 58: control output of the camera control unit 50
- **70**: **rinsing device**
- 71: rinsing fluid reservoir of the rinsing device 70
- 72: rinsing fluid pump of the rinsing device 70
- 73: pressure reservoir of the rinsing device 70
- 75: control input
- 76: pressure sensor of the rinsing device 70
- 77: flow sensor of the rinsing device 70
- 78: sensor signal output of the rinsing device 70
- 79: rinsing fluid output of the rinsing device 70
- 91: vertical blanking interval
- 92: interval between two consecutive vertical blanking intervals 91

- 101: first step (receiving a sensor signal from a sensor detecting a user input starting a rinsing operation)
- 102: second step (changing an illumination mode from a first illumination mode to a second illumination mode)
- 103: third step (changing a capturing mode to a maximum exposure time capturing mode)
- 104: fourth step (rinsing the light entrance surface with a rinsing liquid)
- 105: fifth step (drying the light entrance surface with gas)
- 106: sixth step (receiving a sensor signal from a sensor detecting a user input stopping a rinsing operation)
- 107: seventh step (changing the illumination mode from the second illumination mode to the first illumination mode)
- 108: eighth step (changing the capturing mode from the second capturing mode to the first capturing mode)

## Claims

1. **Method of operating an endoscopic system** (10) comprising a light source (40), an endoscope (20) with a distal light entrance surface (23), an image sensor (25) capturing images through the light entrance surface (23) and a rinsing device (70) providing a rinsing fluid flow for rinsing the light entrance surface (23), the method comprising:
**receiving** (101) a sensor signal from a sensor (31; 35; 36; 37; 76; 77) detecting a user input at a user interface (30), the user input starting a rinsing operation of the rinsing device (70);
upon receipt of the sensor signal, **changing** (102) an illumination mode of the light source (40) from a first illumination mode to a second illumination mode.

2. Method according to the preceding claim, wherein receiving (101) the sensor signal comprises receiving a sensor signal from a sensor (35; 36; 37; 76; 77) detecting a manual operation of a **valve** (33) controlling a flow of the rinsing fluid.

3. Method according to one of the preceding claims, wherein receiving (101) the sensor signal comprises receiving a sensor signal from a **hall** sensor (35) or a switch or another sensor detecting a position of a **valve member** of the valve (33).

4. Method according to one of the preceding claims, wherein receiving (101) the sensor signal comprises receiving a sensor signal from a sensor (36; 37; 76; 77) detecting at least one of a **pressure** or a **flow** or a change of pressure or a change of flow of the rinsing fluid.

5. Method according to one of the preceding claims, wherein receiving the sensor signal comprises receiving (101) the sensor signal from a sensor in the **endoscope** (20) or in the **rinsing device** (70).

6. Method according to one of the preceding claims, wherein
spectral characteristics of illumination light provided by the light source (40) are equal in the first and second illumination light modes,
power or time dependence of power of illumination light provided by the light source (40) is different in the first and second illumination modes.

7. Method according to one of the preceding claims, wherein
in the second illumination mode, power or intensity of light provided by the light source is in particular higher within a vertical blanking interval of the image sensor than outside the vertical blanking interval.

8. Method according to one of the preceding claims, wherein
in the second illumination mode the **intensity** of light provided by the light source (40) is **reduced.**

9. Method according to one of the preceding claims, further comprising:
receiving (106) a further signal indicating an **end of the rinsing operation** from the sensor (35; 36; 37) or from another sensor;
upon receipt of the further signal, changing (107) the illumination mode of the light source (40) from the second illumination mode to the first illumination mode.

10. Method according to the preceding claim, wherein
the rinsing operation comprises a gas flowing at the distal light entrance surface (23) after a rinsing liquid flew at the distal light entrance surface (23),
receiving the further signal comprises receiving (106) a further signal indicating that the gas flow is terminated.

11. Method according to one of the claims 9 and 10, wherein the operating mode of the light source (40) is **gradually** changed back from the second illumination mode to the first illumination mode.

12. **Endoscope** (20) comprising:
a **shaft** (21);
a **light entrance surface** (23) at a distal end (22) of the shaft (21);
a **rinsing fluid duct** (34) in the shaft (21) conducting a rinsing fluid from the proximal end (29) of the endoscope (20) to the light entrance surface (23) for rinsing the light entrance surface (23);
at least one of a **pressure sensor** (36) for the detection of a pressure or a change of pressure in the rinsing fluid duct (34) and a **flow sensor** (37) for the detection of a fluid flow or a change of fluid flow in the rinsing fluid duct (34) and a **user interface sensor** (31; 35) for directly detecting a user input at a user interface (30), the user input requesting rinsing of the light entrance surface (23);
a **sensor signal output** (38) configured to provide a signal representing the pressure or the change of pressure detected by the pressure sensor (36) or the flow or the change of flow detected by the flow sensor (37) or the user input detected by the user interface sensor (31; 35), respectively.

13. **Rinsing device** (70) comprising:
a **pressure source** (72) providing pressure for conveying rinsing fluid;
a **rinsing fluid output** (79) fluidly coupled to the pressure source (72) for providing rinsing fluid to an endoscope (20);
at least one of a **pressure sensor** (76) for the detection of a pressure or a change of pressure of rinsing fluid and a **flow sensor** (77) for detection of a flow or a change of flow of rinsing fluid and a **user interface sensor** (31) for directly detecting a user input at a user interface (30), the user input requesting rinsing of a light entrance surface (23);
a sensor **signal output** (78) configured to provide a signal representing the pressure or the change of pressure detected by the pressure sensor (76) or the flow or the change of flow detected by the flow sensor (77) or the user input detected by the user input sensor (31), respectively.

14. **Camera control unit** (50) comprising:
an **image signal input** (53) for receiving an image signal representing an image captured by an image sensor (25) of an endoscope (20) or a camera coupled to an endoscope (20);
a **sensor signal input** (56) for receiving a sensor signal indicating at least one of a **pressure or a change of pressure or a flow or a change of flow** of a rinsing fluid and a **user input** requesting rinsing of a light entrance surface (23) of an endoscope (20);
a **control output** (58) for providing a control signal **changing** an illumination mode of a light source (40) from a first illumination mode to a second illumination mode when a sensor signal indicating a rinsing operation is received at the sensor signal input (56).

15. **An endoscopic system** (10) comprising:
an **endoscope** (20) with a shaft (21), a light entrance surface (23) at a distal end (22) of the shaft (21) and a rinsing fluid duct (34) in the shaft (21) for conducting a rinsing fluid from the proximal end (29) of the endoscope (20) to the light entrance surface (23) for rinsing the light entrance surface (23);
a **light source** (40) for providing illumination light for the endoscope (20);
at least one of a **pressure sensor** (36; 76) for detection of a pressure or change of pressure of rinsing fluid and a **flow sensor** (37; 77) for detection of a flow or change of flow of rinsing fluid and a user interface sensor (31; 35) for detecting a user input at a user interface (30), the user input requesting rinsing of the light entrance surface (23);
a **controller** (50) coupled to the pressure or flow sensor (36; 37; 76; 77) or user interface sensor (31; 35), respectively, and to the light source (40), for **changing** (102) an illumination mode of the light source (40) from a first illumination mode to a second illumination mode when a sensor signal from the pressure sensor (36; 76) or flow sensor (37; 77) or user input sensor (31; 35), respectively, indicates a rinsing operation.
